Europäisches Patentamt

European Patent Office

Office européen des brevets

(11) Publication number: **0 292 879**
**A2**

(12) # EUROPEAN PATENT APPLICATION

(21) Application number: **88108049.3**

(22) Date of filing: **19.05.88**

(51) Int. Cl.4: **C12N 15/00 , C12N 5/00**

The applicant has filed a statement in accordance with Rule 28 (4) EPC (issue of a sample only to an expert). Accession number(s) of the deposit(s): DSM 4030.

(30) Priority: **22.05.87 US 52827**

(43) Date of publication of application:
**30.11.88 Bulletin 88/48**

(84) Designated Contracting States:
**AT BE CH DE ES FR GB GR IT LI LU NL SE**

(71) Applicant: **ORION CORPORATION LTD.**
**P.O. Box 65**
**SF-02101 Espoo(FI)**

(72) Inventor: **Ismo, Johannes Ulmanen**
**Harmotie 1 F 64**
**SF-01200 Vantaa(FI)**
Inventor: **Anu, Jalanko**
**Anjankuja 3 B 143**
**SF-02230 Espoo(FI)**
Inventor: **Arja, Irmeli Kallio**
**Westendintie 109 B**
**SF-02160 Espoo(FI)**

(74) Representative: **Vossius & Partner**
**Siebertstrasse 4 P.O. Box 86 07 67**
**D-8000 München 86(DE)**

(54) **An episomal vector for the expression of selected DNA fragments coding for polypeptides in mammalian cells and methods for the production thereof.**

(57) The present invention is directed to an episomal expression vector with an efficient expression potential of cloned DNA sequences. The single vector plasmid of this invention joins DNA-fragments, which allow extra chromosomal replication of the vector in a wide selection of the host cells and fragments, which give the transfected cells resistance for a selection drug and a mammalian expression cassette comprising one of the strongest eucaryotic enhancers known at present. The vector also carries appropriate promoter and transcription processing signals. This new combination provides this expression vector with several properties that makes it optimal to be used in transfection of cell lines suitable for production and expression of foreign DNA sequences such as cDNA sequences joined into this vector with high efficiency.

Fig. 1

EP 0 292 879 A2

## AN EPISOMAL VECTOR FOR THE EXPRESSION OF SELECTED DNA FRAGMENTS CODING FOR POLYPEP- TIDES IN MAMMALIAN CELLS AND METHODS FOR THE PRODUCTION THEREOF

The present invention relates to a novel mammalian expression vector comprising an Epstein-Barr-virus-based DNA sequence responsible for extrachromosomal (episomal) replication and a human cytomegalovirus-enhancer for efficient expression of selected DNA sequences coding for polypeptides. The invention also discloses mammalian hosts capable of being transfected by the vector of the present invention and wherein the vector is in extrachromosomal form. The invention discloses methods for preparing the vector and host as well as methods for producing the polypeptides in mammalian cells.

Expression of isolated cDNAs or DNA fragments coding for selected proteins especially proteins of eucaryotic origin in mammalian cells has major advantages over their expression in procaryotic cells. The quality of the polypeptides produced in eucaryotic cells for use in commercial, especially pharmaceutical preparations is better: the polypeptides are free from undesirable proteins of foreign origin, and the polypep tides often have an higher activity and specificity than the corresponding preparations produced in procaryotic cells. Thus, the expression of foreign DNA in transfected eucaryotic cells has been intensely studied. The earliest patents in this field are U.S. Patent Nos. 4,399,216 and 4,634,665, which disclose the production of proteineous material in eucaryotic cells. In these patents, it is specifically emphasized that the foreign DNA must be incorporated into the chromosomal DNA of the eucaryotic cell.

However, later studies showed that the integration of transfected DNA into the host cell chromosomes could not be efficiently targeted. Thus, it was difficult to select and maintain stable transfected cell lines. These difficulties stimulated the study of episomal expression vectors. So far episomal replication of clones DNAs in mammalian cells has been achieved by using, in combination with appropriate cell lines, vectors carrying papillomavirus DNA sequences as disclosed in U.S. Patent No. 4,419,446 and European Patent Application No. 198 386 and Epstein-Barr virus DNA fragments described by Yates et al. in Nature 313:813-815 (1985) and Sudgen et al. in Molecular and Cellular Biology 5(2):410-413 (1985) and Shimizu et al. in Molecular and Cellular Biology 6 (4):1074-1087 (1986) or vectors with DNA replication origins of Simian virus i.e., SV40 described in U.S. Patent No. 4,599,308 or polyoma virus described by Miller et al. in Mol. Cell. Bio. 4(11):2406-2412 (1984).

In order to express cloned sequences in mammalian cells, foreign DNA has to be joined with an expression vector which carries the necessary information for transcription initiation and processing of new tran scripts. Constitutive or regulated expression of cloned cDNA has been reached using several enhancers and promoters of cellular and viral origin. The quality of an enhancer is a major factor regulating the transcriptional activity of a gene and consequently, the amount of the gene product. A CMV-enhancer has been disclosed in the European Patent Application No. 173 522 and a Papovavirus-enhancer in European Patent Application No. 154 566.

This invention provides a novel extrachromosomal vector for high expression of a selected DNA fragment coding for a polypeptide(s) in selected mammalian cells; the vector having the capacity of transfecting mammalian cells and replicating in episomal form, and which vector comprises the following DNA segments:

(a) a regulation sequence for initiation of transcription, wherein the regulation sequence for the initiation of transcription comprises the enhancer sequence of the immediate early genes of the human cytomegalovirus and the promoter of early genes of the SV40 virus;

(b) a sequence comprising of the processing signals for synthetized mRNA, wherein the sequence comprising the processing signals for synthetized mRNA consists of the small t-antigen intron and the polyadenylation signals of the early genes of the SV40 virus;

(c) a DNA sequence responsible for the extrachromosomal replication of the vector, wherein the DNA sequence responsible for the extrachromosomal replica tion of the vector comprises the EBNA-1 gene and the ori-P sequence of the Epstein-Barr virus;

(d) a multiple cloning site, comprising three unique restriction enzyme cleavage sites, HindIII, SalI and XbaI, whereto the DNA sequence coding for the selected polypeptides are ligated;

(e) at least one optional, suitable marker sequence for selection in bacteria, wherein the optional, suitable marker for selection in bacteria is the amp$^r$ gene of the pBR322 plasmid and the initiation site of the DNA replication in pBR322; and

(f) at least one optional, suitable marker gene for selection in mammalian cells wherein the gene for the selection in mammalian cells is the hph-gene from transposon Tn5 of E. coli, which gene confers resistance to hygromycin B.

The plasmid pKTH539 which comprises the segments mentioned above has been deposited in the culture collection Deutsche Sammlung von Microorganismen and has deposit accession number DSM 4030.

This invention also provides a mammalian host selected from a group comprising human HeLa cells, canine MDCK, monkey CV-1, hamster R1610, human 293 and human Sultan cells, capable of being transfected with at least one vector of the present invention.

The present invention also provides methods for producing in mammalian cells, selected polypeptides, such as bacterial chloramphenicol acetyl transferase (CAT), influenza virus hemagglutinin, rubella virus polypeptides, tissue plasminogen activator (TPA), Factor VIII or von Willebrandt factor or antigens of the HI-virus, Hepatitis D virus. The method comprises the steps of preparing the episomal vector of the present invention and culturing a host selected from a group comprising human HeLa cells, canine MDCK, monkey CV-1, hamster R1610, human 293 and human Sultan cells, transfected with at least one episomal vector of the present invention.

The figures show:

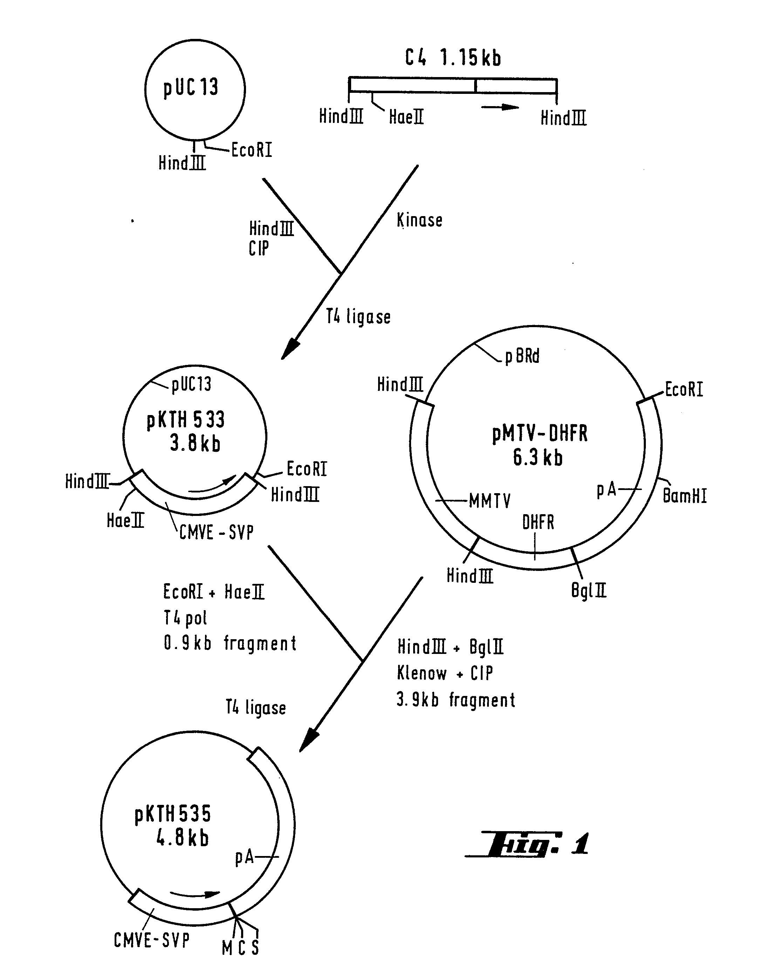

Fig. 1: Construction of recombinant plasmid pKTH535. The recombinant plasmid pKTH535 was constructed by first subcloning the CMV C4 HindIII fragment to pUC13 at the HindIII site. From the pUC13 polylinker, the BamHI cloning site was omitted with BamHI digestion, filled in with Klenow and religated. From this subclone (pKTH533) the EcoRI-HaeII fragment which contained the CMV enhancer, SV40 promoter and pUC13 polylinker was isolated, blunt-ended with T4 polymerase and ligated to a blunt-ended pMTV-dhfr BglII-HindIII fragment, which contained the SV40 intron and polyadenylation site and pBRd.

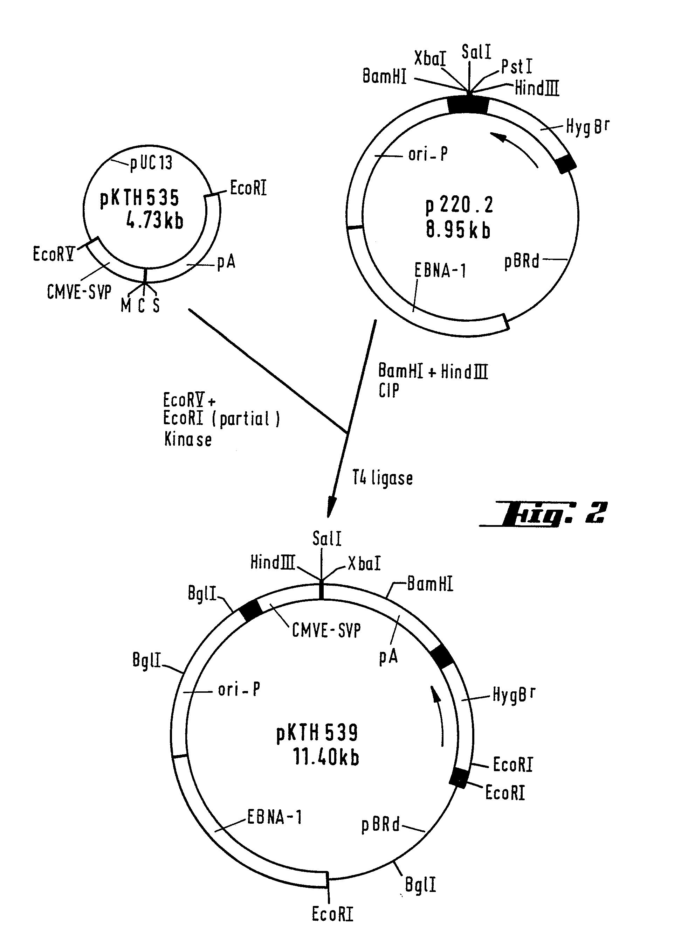

Fig. 2: Construction of recombinant plasmid pKTH539. The EBV vector pKTH539 was constructed from pKTH535 and p220.2. The plasmid p220.2 was digested with BamHI and HindIII, filled in with Klenow enzyme and ligated with the 2.1 kb EcoRV-EcRI fragment of pKTH535 that had been blunt-ended with Klenow.

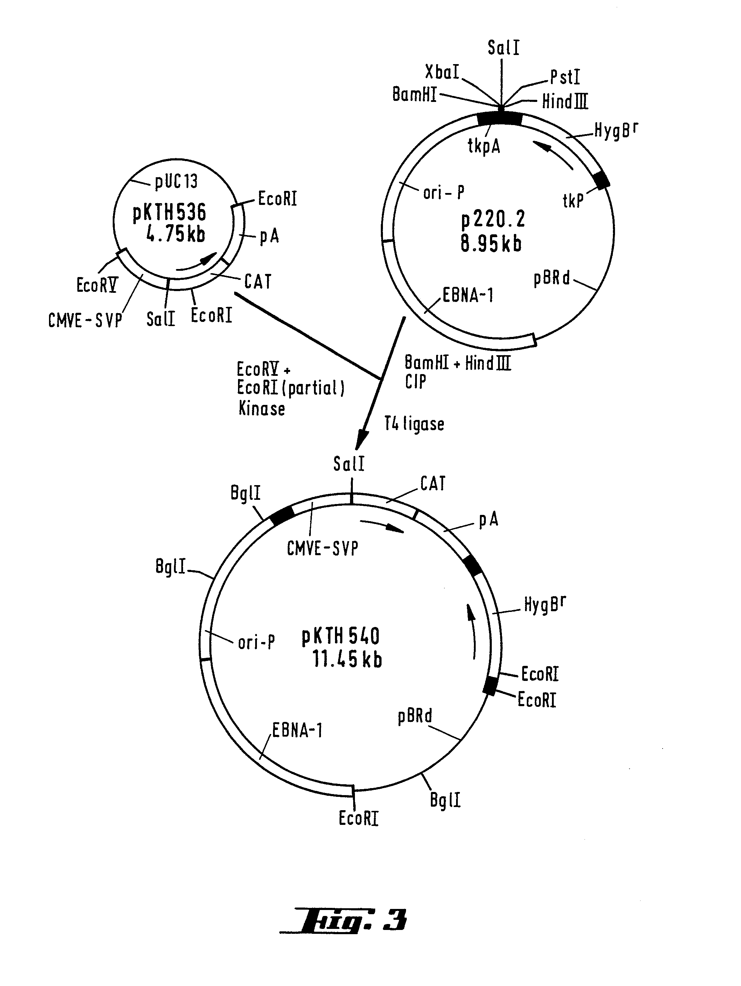

Fig. 3: Construction of recombinant plasmid pKTH540 i.e. the CAT-expressing vector. The recombinant plasmid pKTH540 was constructed by first inserting a blunt-ended HindIII-BamHI fragment of pSV2CAT into the CMV-pUC13 subclone (pKTH533) SmaI site. This pCMV-CAT subclone (pKTH536) was then cleaved with EcoRV-EcoRI (partial EcoRI) digest to get the 2.1 kb CMV-SV40-polylinker-CAT-polyA fragment which was bluntended and inserted into p220.2 HindIII-BamHI fragment (Klenow-filled) to make pKTH540.

The present invention is directed to an episomal expression vector with an efficient expression potential of cloned DNA sequences. The vector plasmid of this invention joins DNA-fragments, which allow extrachromosomal replication of the vector in a wide selection of host cells and fragments, which give the transfected cells resistance for a selection drug and a mammalian expression cassette comprising one of the strongest eucaryotic enhancers known at present. The vector also carries appropriate promoter and transcription processing signals. This new combination provides this expression vector with several properties that makes it optimal to be used in transfection of cell lines suitable for production and expression of foreign cDNAs joined into this vector with high efficiency. The combination of a high expression capacity with extrachromosomal replication system as applied in this vector, has the following advantages compared with previous methods:

1) High transfection efficiency giving significantly higher amounts of transfected cell clones that comparable integrating vectors;

2) Low amount of rearrangements, which could inactivate the expression of cloned cDNA;

3) High plasmid stability upon extended maintenance of cell clones;

4) Efficient expression of cloned sequences from the extrachromosomal vector in a high proportion of transfected cells.

A more complete understanding can be obtained by reference to the following specific examples which are provided herein for purpose of illustration only and are not intended to be limiting unless otherwise specified.

## EXAMPLE 1

The construction and the use of an episomal vector for the expression of a DNA fragment coding for the chloramphenicol acetyl transferase (CAT).

## MATERIALS AND METHODS

(a) Vectors and bacterial strains

pSV2CAT was obtained from B. Howard (Cerman et al., Mol. Cell. Biol. 2:1044-1051 (1982)), p220.2 from Bill Sudgen, Univ. of Wisconsin, Madison, U.S.A., and pMTV dhfr from G. Ringold (Lee et al. Natur 294:228-232 (1981)). The cytomegalovirus enhancer was obtained from the SV40-CMV clone C4 (M. Boshart, Cell 41:521-530 (1985)). pHA1 was obtained from M.J. Gething (Gething et al., Nature 287:301-306 (1980)). E. coli B2 was used as a host for other vectors than pUC13 and M13mp11, for which the E. coli JM103 was the host.

(b) Cell culture and transfections

Hela cells (human cervical carcinoma, ATCC CCL 2), MDCK cells (canine kidney, ATCC CCL 34), CV-1 cells (African green monkey kidney, ATCC CCL 70), R1610 (chinese hamster somatic cells, from D. Picard, Univ. of Zurich, Switzerland), and 293 cells (transformed primary human embryonal kidney, from U. Petterson, Univ. of Uppsala, Sweden), were cultured in MEM supplemented with penicillin-streptomycin and 10% fetal calf serum. After transfection and selection, the serum level was lowered to 5%. Sultan cells (human myeloma, cell line, P. Leinonen, Univ. of Helsinki, Finland) were cultured in suspension in MEM supplemented with penicillinstreptomycin, 10% fetal calf serum and 10 mN Hepes, pH 7.2.

DNA was transfected to cell monolayers (HeLa, MDCK, CV-1, R 1610 and 293 cells) using a modification of the calcium phosphate coprecipitation technique of Wigler et al. (Cell 14:725-731 1978)) and Weber et al. (Cell 36:983-992 (1984)). One day prior to transfection, the cells were seeded to a 10 cm dish, and the medium was changed 4 h prior to transfection. 20 $\mu$g of ethanol precipitated DNA was dissolved in 240 $\mu$l of $H_2O$, 240 $\mu$l of 4 x Ca (0.5 M $CaCl_2$, 0.1 M Hepes, pH 7.05) was added, incubated for 5 min. at room temperature, and then 480 $\mu$l of 2 x HBS (0.05 M Hepes, pH 7.05, 0.28 M NaCl, 0.75 mM $Na_2PO_4$ and 0.75 mM $NaH_2PO_4$) was added. After 15 min. incubation at room temperature, the calcium phosphate-DNA coprecipitate was added to the cell monolayers and incubated at 37° C for 12 h. The cells were washed with TBS (25 mM Tris, pH 7.4, 137 mM NaCl, 5 mM KCl, 0.7 mM $CaCl_2$, 0.5 mM $MgCl_2$, 0.6 mM $Na_2HPO_4$) and shocked with 25% glycerol for 1 min., washed again twice with TBS and overlayed with fresh medium. After incubation of 24 hrs. at 37° C, the medium as replaced with fresh medium containing 300 $\mu$g/ml of hygromycin B. Resistant colonies appeared 10-14 days after transfection, and they were collected by cloning rings or by making single cell clones from the pooled cells. The transformation efficiency varied between 1 to 100 colonies per $\mu$g of DNA for 2.5 to 5 x $10^5$ cells.

The Sultan suspension cells were transfected by electroporation according to Neumann et al., EMBO J. 1:841-843 (1982) and Potter et al., PNAS 81:7161-7165 (1984). The electroporation was done in 200 $\mu$l of Hepes buffered saline (140 mM NaCl, 25 mM Hepes, 0.75 mM $Na_2HPO_4$, pH 7.1) with 2 x $10^6$ cells and 12 $\mu$g of DNA. The cell-DNA suspension was exposed to 3 pulses at 7 second intervals. The electrical field was 5 V/cm with 88 nF. After the electroporation, the cells were incubated on ice for 20 min. and transferred to cell culture flasks with medium.

(c) Isolation and analysis of DNA

Total cellular DNA was isolated from transfected cells by a modification of the method described by Maniatis et al. (Molecular Cloning, A Laboratory Manual, Cold Spring Harbor Laboratory. N.Y. 1982) Specifically, 5 x $10^6$ cells were resuspended in 50 $\mu$l of TE-buffer, 500 $\mu$l of proteinase K buffer (0.1 M NaCl, 0.05 M Tris, pH 8.0, 0.02 M EDTA and 0.5 % SDS) was added and incubated with 100 $\mu$g/ml of proteinase K for 2 h at 37° C. The lysate was then phenol extracted twice and ethanol precipitated. The pellet was dissolved in 300 $\mu$l of TEX buffer (50 mMTris, pH 8, 10 mM EDTA, 10 mM NaCl) and incubated with RNAse (100 $\mu$g/l) for 2 h at 37° C, phenol extracted and ethanol precipitated, which resulted in 20 ug of total DNA, which was then run in 0.8 % agarose gel electrophoresis in Tris-acetate buffer. Blotting and hybridization was according to Maniatis et al. (1982). Probe DNA was nick-translated pKTH540 with a specific activity of $18^8$ cpm/$\mu$g.

<u>(d) CAT assay</u>

The cells from stable transfected pools or clones were harvested generally at 48 h after seeding. The assay method of Gorman <u>et al.</u>, (1982) was used. The CAT enzyme was released from $10^6$ cells into 100 $\mu$l of 250 mM Tris-HCl (pH 7.8) by freezing and thawing three times. The enzyme assay was done in a final volume of 150 $\mu$l of 500 mM Tris-HCl (pH 7.8) containing 10 $\mu$l of the cell extract, 20 $\mu$l of 4 mM acetyl coenzyme A (Pharmacia) and 0.5 $\mu$Ci of $^{14}$C chloramphenicol (54 mCi/mmole; Amersham). To quantitate CAT activity, standard curves were generated using known amounts of purified CAT enzyme (Pharmacia). The enzyme assay was run for 30 min at 37° C and stopped by extracting the chloramphenicol with 0.8 ml of ethyl acetate. After centrifugal evaporation the chloramphenicol was dissolved in 15 $\mu$l of ethyl acetate, spotted on silica gel thin layer plates (Merck) and run with chloroform-methanol (95:5, ascending). After autoradiography, the amount of acetylated chloramphenicol was quantitated by cutting the spots out and counting them in a scintillation counter. Sample extracts were diluted for CAT assay, if the percentage of acetylation exceeded 30% indicating substrate exhaustion. Values of percent acetylation within the linear range were converted to CAT activities in units.

<u>(e) Sandwich hybridization</u>

For the accurate determination of copy numbers of transferred DNA, a sandwich hybridization assay was used as described in UK Patent Publication GB 2187283.

<div align="center">

CONSTRUCTION OF EPISOMAL RECOMBINANT PLASMIDS

</div>

The cytomegalovirus enhancer and SV40 promoter were chosen for the regulatory sequences of the expression vector, because the CMV enhancer is the strongest known enhancer with a wide host cell range. The 1.15 kb CMV enhancer - SV40 promoter fragment was obtained from the plasmid CMV C4 by HindIII digestion (Fig. 1). The HindIII fragment was isolated and subcloned to pUC13 vector at the HindIII site (Fig. 1). From the pUC13 polylinker cloning site, the BamHI site was omitted with BamHI digest, Klenow fill in and religation. From this subclone (pKTH533), the CMV enhancer - SV40 promoter and the pUC13 polyliner containing 0.9 kb EcoRI-HaeII fragment was isolated, blunt-ended with T$_4$ polymerase and ligated to a blunt-ended pMTV-dhfr BglII-HindIII fragment (3.9 kb) which contained the SV40 polyadenylation site and the pBRd sequence (Fig. 1). The resulting integrating vector pKTH535 now contained a polylinker cloning site with unique XbaI and SalI sites.

The episomal EBV vector pKTH539 (Fig. 2) was constructed from pKTH535 (Fig. 2) which contains the CMV enhancer, SV40 promoter, polylinker cloning site and polyadenylation signal. The eBV fragments EBNA-1 and ori-P that are needed for episomal replication, were obtained from plasmid p220.2 (Fig. 2). This plasmid also contains a hygromycin B resistancy gen <u>hph</u> for selection of transformants. p220.2 was digested with BamHI and HindIII, filled in with Klenow enzyme (this removes the XbaI, SalI and PstI sites from p220.2) and ligated with the 2.1 kb EcoRV-EcoRI fragment of pKTH535 that had been blunt-ended with Klenow (Fig. 2). pKTH539 has unique HindIII, XbaI and SalI sites for cloning.

The CAT-EBV vector recombinant pKTH 540 (Fig. 3) was constructed by first inserting a HindIII-BamHI fragment of pSV2CAT into the CMV-pUC13 subclone pKTH53 at the SmaI site. This pCMVCAT subclone (pKTH536) was used together with pSV2CAT in the transient promoter activity assays. To ligate the EBV-HygB$^r$ fragment, pKTH536 was cleaved with EcoRV-EcoRI (partial EcoRI) to get the 2.1 kb CMV enhancer-SV40-promoter-polylinker-poly(A)-fragment. This fragment was isolated, blunt-ended and inserted into Klenow-filled p220.2 HindIII-BamHI fragment to obtain pKTH540 (Fig. 3).

<div align="center">

EXPERIMENTAL

</div>

<div align="center">

5

</div>

## (a) Analysis of vector DNA in transfected cells

The state of the vector pKTH540 DNA in transfected cell pools was analyzed by isolating total cellular DNA and analyzing the purified DNAs after light mechanical shearing by agarose gel electrophoresis. Southern blots of the nondigested DNAs from HeLa, CV-1 and 293 cells, probed with plasmid specific probes, revealed a signal that comigrated with the monomeric pKTH540 marker DNA, which indicates that in these cells the transfected DNA resides in episomal form. In R1610 and MDCK cells, the hybridizing DNA in the non-digested samples was detected at the top of the gels thus comigrating with the chromosomal host DNA.

This range of cells supplying episomal replication of EBV vectors confirms the observations made by Yates et all, Nature 313:812-815 (1985). According to our and others' experience, a BPV-based vector is in episomal form in certain rodent cells (C127 and NIH3T3), but integrates in cells of several other mammalian species (Ruohonen-Lehto et al., J. Biotechnology 6: 91-105, 1987). Thus, EBV- and BPV-vectors seem to complement each other regarding the host spectrum of the episomality.

## (b) Comparison of CMV and SV40 enhancer activities by transient expression assay

CMV enhancer has been found to be several fold more active than SV40 early enhancer when inserted into SV40 virus genome (Boshart, 1985). We wanted to verify if this difference is expressed in expression vector constructions when these promoters direct the synthesis of cloned genes. For this purpose a CAT coding sequence was inserted into a vector carrying CMV-SV40 enhancer promoter and transcription processing sequences (pKTH535 in Fig. 1). The resulting plasmid, CMV-CAT, was transfected by calcium phosphate precipitation into various cultured cells, and CAT activities were determined 48 h after transfection. As a comparison, the same amount of cells was transfected with pSVCAT plasmid, where SV40 early enhancer promoter reside in front of the CAT gene (Gorman et al., 1982). Table 1 shows that in rodent and monkey cells the CMV enhancer is 1.3-4.7 times more active than the SV40 enhancer, whereas in human cells SV40 enhancer gives slightly higher CAT activities. These results indicate that CMV enhancer is indeed a promising component of a strong expression vector suitable for use in several types of mammalian cells.

## TABLE 1

### COMPARISON OF RELATIVE PROMOTER ACTIVITIES OF SV-40 EARLY AND CMV-ENHANCER – PROMOTERS IN TRANSIENT CAT-ASSAYS

| CELL TYPE | | PROMOTER ACTIVITY[1] |
|---|---|---|
| HeLa | human cervical carcinoma | 0.6 |
| CV-1 | green monkey kidney | 1.3 |
| R 1610 | chinese hamster cells | 2.8 |
| BHK-21 | hamster kidney | 2.3 |
| L6J1 | rat myoblasts | 4.7 |

1) Ratio of CMV-CAT/SV-CAT cpm in acetylated C14-chloramphenicol

6

### (c) Copy numbers of vectors

Copy numbers of the pKTH540 vector with the CAT gene were determined from the different host cell lines by a sandwich hybridization method. The DNA reagents for hybridization were constructed so that a 3.5 kb EcoRI-BglI fragment of p220.2 containing the EBNA-1 and 1040 bp of ori-P sequences was filled in and ligated into Klenow-treated pBR322-EcoRI digested DNA. This was the probe reagent. The filter reagent for sandwich hybridization was a 1090 bp BglI fragment from p220.2, containing the ori-P sequences downstream from the probe reagent. This fragment was inserted into M13mp11 at the SmaI site. The filter reagent was attached to nitrocellulose filter, and the number of pKTH540 from $3 \times 10^6$ cells were determined.

The copy numbers of plasmid pKTH540 in the hygromycin B selected cell pools and single cell clones are shown in Table 2. Different cell types varied distinctly in their copy numbers of the transfected plasmid. Thus, highest copy numbers were constantly detected in CV-1 cells (6-16 copies/cell) and in 293 cells (1-10 copies/cell). In HeLa, MDCK and R1610 cells most clones had only one copy per cell. However, in these cells, individual clones with high copy number were detected (Table 2). In several clones of HeLa, MDCK and R1610 cells the apparent copy numbers were less than one copy/cell. Based on our earlier data (Ruohonen-Lehto et al., J. Biotechnology 6 : 91-105, 1987), this is the indication of deletion of the plasmid in the regions homologous to the probe and/or filter reagents.

### (d) Expression of CAT enzyme from the CMV-EBV vector

To assay the expression potential of the CMV-EBV vector construct, we cloned the bacterial CAT gene into the vector (Fig. 3) and assayed the CAT activity from the transfected cell pools and cell clones. As shown in Table 2, CAT activity is high in 293, HeLa and CV-1 cells in the cell pools and clones, whereas only one out of 5 cell clones of MDCK, and the pool and one clone of R1610 cells revealed considerable CAT expression.

Inspection of Table 2 shows that there is a positive correlation between the copy numbers and the CAT activities. In 293 and CV-1 cells, the episomal vector occurred in most cell clones as more than one copy numbers. In HeLa cells, the CAT activities were remarkably high, but the correlation with the copy numbers was less clear. However, clone number 4, with the highest CAT activity (193 U/$10^7$ cells) also carries about 20 vector copies/cell.

The poor expression of CAT gene in most MDCK and R1610 cell clones could be due to the low copy numbers of the vector DNA. However, MDCK clone 5 with one copy/cell shows reasonably high CAT activity, whereas clone 4 with 27 copies/cell has a very low enzyme activity. The reason that most MDCK and R1610 clones have apparent copy numbers less than one, together with the Southern hybridization results, suggests that in these cells the transfected DNA has integrated into the host genome. This integration process apparently strongly affects the integrity of the vector DNA and caused in high frequence a decreased activity of transfected genes. Our results show that the expression of cloned marker gene in the CMV-EBV vector is effective particularly in those cells where it replicates in episomal form. The fact that most episomal cell clones showed high CAT expression supports the assumption that cloned gene in episomal vector undergoes less rearrangements and is not subject to cis-acting down-regulation by flanking chromosomal region in integration site. Thus, EBV-vectors can be efficiently applied for isolation of chromosomal genes and they also provide a promising vector for high production of foreign eukaryotic proteins in mammalian cells.

### (e) Expression of CAT gene from episomal versus integrated CMV vectors

The determination of CAT activities from R1610 and MDCK cells, where CMV-EBV vector integrated, already suggested that expression might on the average be lower in these cells than in human and monkey cells where the vector remained episomal (see Table 2). To verify this assumption, we cotransfected into human and monkey cells (and also into dog and rodent cells) integrating CMV-CAT construct (pKTH536 in Fig. 3) with a vector which confers the cells resistant to selection with hygromycin B. We selected the resistant cell pools and five random single cell clones from each cell type. Determination of the CAT activities from these cells strongly supported the view that episomal genes are on the average more active. The inspection of CAT activities in single cell clones supported the view that variation in the levels of expression of cloned genes in integrating vectors is significantly higher than from episomal vectors. Several reasons may contribute for this difference observed. Certainly one factor is the frequent structural changes occurring in the integrating DNA sequences in the cell nuclei. Also the regulation exerted by the chromosomal region of integration site can inhibit the transcriptional activity of otherwise intact gene.

## TABLE 2

### CHLORAMPHENICOL ACETYL TRANSFERASE (CAT) ACTIVITY IN pKTH 540 (CMV-EBV-CAT) TRANSFORMED CELLS

| CELLS | CAT ACTIVITY (UNITS/$10^7$ CELLS)[1] | COPY NUMBER |
|---|---|---|
| HUMAN 293 POOL | 8.3 | 2.0 |
| 1 | 8.8 | 3.0 |
| 2 | 4.6 | 1.0 |
| 3 | 16.8 | 10.0 |
| 4 | 7.6 | 5.0 |
| 5 | 16.2 | 5.0 |
| HUMAN HELA POOL | 34.0 | 1.0 |
| 1 | 41.0 | <1 |
| 2 | 8.0 | <1 |
| 3 | 6.6 | 1.0 |
| 4 | 193.0 | 20.0 |
| 5 | 29.0 | <1 |
| MONKEY CV-1 POOL | 23.0 | 12.0 |
| 1 | 9.2 | 13.0 |
| 2 | 7.2 | 10.0 |
| 3 | 6.6 | 13.0 |
| 4 | 3.4 | 6.0 |
| 5 | 40.0 | 16.7 |
| DOG MDCK POOL | 0.8 | 2.0 |
| 1 | 0.3 | <1 |
| 2 | 0.4 | <1 |
| 3 | 1.0 | <1 |
| 4 | 0.2 | 26.6 |
| 5 | 9.4 | 1.0 |
| HAMSTER R1610 POOL | 9.7 | 5.0 |
| 1 | 0.4 | <1 |
| 2 | 30.2 | 18.0 |
| 3 | 0.6 | <1 |
| 4 | 0.4 | <1 |
| 5 | 0.4 | <1 |

1) Commercial _E. coli_ CAT was used as control. One unit of enzyme acetylates one nanomol of chloramphenicol in one minute at 37°C.

(f) Stability of CMV EBV vector in transfected cell clones

It is of great interest to know how stable are the episomal vector sequences and genes cloned in these vectors in transfected cells. Of particular practical importance is the question of whether the cells must be kept under constant selection pressure. Earlier results gave conflicting information; in some cases of BPV vectors in rodent cells episomal sequences were found to remain seemingly intact for long periods of time after the release of selection pressure. On the other hand, one report on the expression of HLA-2 gene in EBV vector found that the transfected sequences were rapidly lost when the cells were grown without hygromycin B. We tested the stability of our CMV-EBV vector by cultivating different types of cells transfected by CMV-EBV-CAT construct (pools of hygromycin resistant cells) with and without the drug in the medium. The cells were kept in semiconfluency by seeding them repeatedly about once a week. After 100 days of cultivation the cells were analyzed for the CAT activity and for the number of vector DNA copies (Table 3). In 293, CV-1 and R1610 cells the vector sequences were apparently lost in the absence of hygromycin B selection, as indicated by decrease in copy numbers. Significantly, in these cells the CAT activities also dropped dramatically, although activity was clearly detectable. The absence of selection did not affect the copy numbers of the vector in HeLa of MDCK cells and in these cells the CAT activities consequently remained unchanged.

## TABLE 3

### STABILITY OF pKTH540

Stability of pKTH 540 sequences and CAT expression in transfected cell pools after 100 days of cultivation of cells with and without hygromycin B selection.

| Cell type | With HygB | | Without HygB | |
|---|---|---|---|---|
| | Copies[1] | CAT activity[2] | Copies | CAT activity |
| 293 | 2 | 36.6 | <1 | 3.2 |
| HeLa | 1 | 27.4 | 1 | 40.9 |
| CV-1 | 12 | 55.9 | <1 | 6.0 |
| MDCK | 2 | 2.7 | 3 | 2.2 |
| R1610 | 5 | 24.6 | <1 | 2.5 |

1) Number of plasmid copies/cell, determined by sandwich hybridization.

2) Activity of chloramphenicol acetytransferase enzyme, units per $10^7$ cells.

## EXAMPLE 2

The construction and use of an episomal vector for the expression of a DNA fragment coding for the influenza virus hemagglutinin.

The influenza virus hemagglutinin (HA) DNA was excised from plasmid pHD1 with HindIII, BamHI digestion, filled with the Klenow enzyme, isolated after agarose gel electrophoresis and inserted into the Klenow filled pKTH539 HindIII site. This EBV-HA construct containing the HA cDNA under the control of CMV enhancer-SV40 promoter was named pKTH541.

9

## MATERIALS AND METHODS

### Immunofluorescence staining

For immunofluorescence staining the stable cell pools of HeLa/pKTH541 and293/pKTH541 were fixed with 3% paraformaldehyde. Indirect immunofluorescence was performed with rabbit (anti $H_2N_2$, influenza virus Jap.) (I. Julkunen, Dept. Virology, Univ. of Helsinki, Finland) and rhodamine-conjugated anti-rabbit IgC.

## RESULTS

### Expression of HA gene from EBV vector

The stable transfected HeLa1541 and 293/541 cell pools were analyzed for the presence of HA surface glycoprotein by indirect immunofluorescence staining using influenza virus antiserum. Both cell pools shows extensive staining of HA on the surface of cells indicating that the EBV vector also is functional when expressing cell surface proteins.

The invention now being fully described, it will be apparent to one of ordinary skill in the art that many changes and modifications can be made thereto without departing from the spirit or scope of the invention as set forth herein.

## Claims

1. An episomal vector for high expression of a selected DNA fragment coding for a polypeptide in selected mammalian cells, the vector having the capacity of transfecting mammalian cells and replicating in extrachromosomal form, and which vector comprises the following DNA segments:

(a) a regulation sequence for initiation of transcription, comprising the enhancer sequence of the immediate early genes of the human cytomegalovirus and the promoter of early genes of the SV40 virus or any functional parts thereof;

(b) a sequence comprising the processing signals for synthesized mRNA comprising the small t-antigen intron and the polyadenylation signals of the early genes of the SV40 virus or any functional part thereof;

(c) a DNA sequence responsible for the extrachromosomal replication of the vector comprising the EBNA-1 gene and the ori-P sequence of the Epstein-Barr virus and any functional groups thereof;

(d) at least one cloning site and preferably a multiple cloning site comprising at least one unique restriction enzyme cleavage site into which the selected DNA fragment coding for the desired polypeptide can be ligated; optionally

(e) a suitable marker sequence for selection in bacteria; and optionally

(f) a suitable marker gene for selection in mammalian cells.

2. The vector according to claim 1, wherein the multiple cloning site is derived from the plasmid pUC13 and comprises restriction sites for the three restriction enzymes HindIII, SalI, and XbaI.

3. The vector of claim 1 or 2 wherein the suitable marker for selection in bacteria is the amp$^r$ gene of plasmid pBR322 and the initiation site of the DNA replication of pBR322.

4. The vector of any one of claims 1 to 3, wherein the suitable marker gene for the selection in mammalian cells is the hph-gene from transposon Tn5 of E. coli, which confers resistance to hygromycin B.

5. Vector pKTH 539 (DSM 4030).

6. A recombinant vector comprising a vector according to any one of claims 1 to 5 and a selected DNA fragment coding for a desired polypeptide which is inserted into said cloning site or multiple cloning site.

7. The vector of claim 6, wherein the selected DNA fragment coding for the desired polypeptide codes for the chloramphenicol acetyl transferase (CAT).

8. The vector of claim 6, wherein the selected DNA fragment coding for the desired polypeptide codes for the influenza virus hemagglutinin.

9. A mammalian host organism containing at least one vector according to any one of claims 1 to 8, which host allows the replication of said vector in extrachromosomal form, and which host is capable of expressing a selected DNA fragment contained in said vector encoding a desired polypeptide.

10. The host according to claim 9, which is a human HeLa cell, a human 293 cell, a human Sultan cell, a monkey CV-1 cell, a canine MDCK cell or a hamster R1610 cell.

11. A method for producing a desired polypeptide in mammalian cells comprising the steps of culturing a mammalian host organism according to claim 9 or 10 under suitable culture conditions and of recovering the polypeptide.

C4 1.15kb

HindⅢ   HaeⅡ          HindⅢ

pUC13
HindⅢ   EcoRI

HindⅢ
CIP          Kinase

T4 ligase

pUC13
pKTH533
3.8kb

HindⅢ          EcoRI
HaeⅡ   CMVE-SVP   HindⅢ

pBRd
HindⅢ          EcoRI
pMTV-DHFR
6.3kb
MMTV      pA   BamHI
DHFR
HindⅢ   BglⅡ

EcoRI + HaeⅡ
T4 pol
0.9kb fragment

HindⅢ + BglⅡ
Klenow + CIP
3.9kb fragment

T4 ligase

pKTH535
4.8kb
pA
CMVE-SVP
MCS

Fig. 1

_Fig. 2_

Fig. 3